# EUROPEAN PATENT APPLICATION

(11) **EP 4 623 841 A1**
(43) Date of publication of application: **01.10.2025**
(21) Application number: 25165795.3
(22) Date of filing: 24.03.2025
(51) Int. Cl.: A61B 17/15

(54) **DEVICES AND METHODS FOR UNICONDYLAR TIBIAL IMPLANTS**

(30) Priority: 27.03.2024 US 202463570527 P
(71) Applicant: Zimmer, Inc., Warsaw, IN 46580 (US)
(72) Inventor: TRISCHLER, Cory, Columbia City, 46725 (US); KREBS, Robert Douglas, Warsaw, 46580 (US)
(74) Representative: Taor, Simon Edward William

(57) **Abstract**

A tibial implant cutting guide comprises a plate to engage a resected surface and an elongate slot extending through the plate along an axis and having first and second longitudinal walls extending parallel to the axis, and a first end wall perpendicular to the axis, the first and second longitudinal walls are spaced apart by a distance and the first end wall is at least as wide as the distance. A method of arthroplasty comprises resecting a tibia to form a resected surface, advancing a cutting head in a cutting slot of a plate to engage flush with a flat first end of the cutting slot to form a bone channel, and inserting a keel into the bone channel. A method of manufacturing a tibial template comprises forming a longitudinal cutting slot in a plate and forming a widening of the cutting slot at an end of the cutting slot.

## Description

### TECHNICAL FIELD

The present disclosure relates generally, by not by way of limitation, to devices and methods for use in performing knee arthroplasty, such as total or partial knee replacement procedures. More specifically, but not by way of limitation, the devices and methods of the present disclosure can be used to perform slot cuts within resected bone surfaces.

### BACKGROUND

A knee joint comprises a femur having two distal convex condyles that engage with two concave condyles at a proximal end of a tibia. The condylar pairings can each form a compartment within the knee joint. Osteoarthritis can develop in a knee joint where cartilage in the knee joint can begin to deteriorate and ultimately fail, leading to bone-on-bone contact and the onset of pain. Osteoarthritis can form in one or both of the compartments. Total knee replacement is the most common surgical treatment for osteoarthritis, involving replacement of the articular surfaces of the femur and tibia in both compartments. Partial knee replacement involves replacement of the articular surfaces in only one compartment of the knee joint, leaving intact the surfaces of the other compartment and all of the ligaments. Partial knee replacement can act prophylactically, reducing the rate of development of the disease in the other compartment. Partial knee replacement can be surgically more demanding due to the potential for having to perform additional bone modifications and the ability to preserve all of the ligaments. Partial knee replacement can be referred to as unicompartmental knee replacement or unicondylar knee replacement.

A femoral or tibial component that is to be implanted into bone can benefit from some manner of fixation to the modified bone surfaces. To this end, prosthetic components often include one or more bone-engaging members, such as anchors, extensions, keels, stems, fins and the like, to project into bone matter to facilitate anchoring of the prosthetic component to the bone. The bone-engaging member can be inserted into a bone cavity that can be produced in a resected bone surface. For example, bores, holes, slots, channels and the like can be formed by drilling, chiseling, reaming, broaching, burring and the like.

Some tibial components utilize a keel, which is an elongate protrusion extending from the base of a tibial plate that extends generally parallel to an anterior-posterior axis of the component. In order to increase the effectiveness of the prosthetic implant and to provide desirable user outcomes, it is advantageous for the surfaces and edges of the prosthetic implant to mate in a desirable fashion with the surfaces and edges of the modified bone. As such, there is a continuing need for instruments used to modify that provide accurate (e.g., in the desired location) and precise (e.g., repeatable) bone modifications.

Examples, of unicondylar or partial knee replacement systems are described in WO 2011/110865 A2 to Goodfellow et al., titled "Tibial prosthetic component for a partial or unicondylar bearing knee replacement, method of selecting such a tibial prosthetic component, method of implanting such a tibial prosthetic component and a kit for a surgeon;" WO 2015/155505 A1 to Dodd et al., titled "Prosthesis with fixed or mobile bearing;" and Pub. No. WO 2022/148964 A2 to O'connor et al., titled "Unicompartmental tibial components."

### OVERVIEW

The present inventors have recognized, among other things, that problems to be solved with traditional partial knee arthroplasties involve the insertion of a keel into a bone slot formed in a proximally resected surface of a tibia. It is desirable for the keel to fit within the bone slot in a secure and accurate manner in the proper location. In particular, it is desirable for the width of the slot to be wide enough to allow the keel to fit in a press-fit configuration for cementless applications to secure the keel in place. Furthermore, it can be advantageous for the keel to be accurately positioned in an anterior-posterior direction for numerous reasons. For example, the tibial component should substantially align with the femoral component of the femoral bone. Furthermore, the present inventors have recognized that imprecise placement of the keel in the anterior-posterior direction can lead to undesirable contact of the keel with the cortical bone wall of the tibia. Specifically, the posterior end of the keel can contact the cortical wall and cause undesirable stress within the cortical bone wall. In extreme cases, the stress can lead to fracture.

The present inventors have also recognized that it can be difficult to obtain precise anterior-posterior placement of the tibial keel due to interaction between the cutting device and a cutting guide slot. For example, the present inventors have recognized that typical cutting guide slots comprise a longitudinal slot that extends in the anterior-posterior direction and that has ends that are rounded. For example, machining process used to form the cutting guide slots can result in curvatures similar to fillets at the ends of the slots. The curved surfaces can interfere with cutting devices inserted therein, e.g., reciprocating saw blade heads, thereby resulting in imprecise placement of the slot.

The present subject matter can provide solutions to these and other problems, such as by providing a cutting guide slot having end surfaces that are ninety-degrees to the walls of the main longitudinal slot. The end surfaces of the cutting guide slot can be formed by machining short perpendicular slots at the end of the longitudinal slot. The short perpendicular slots can form T-shaped ends at the end of the longitudinal slot. The T-shaped ends can form end surfaces at ninety-degrees to the longitudinal slot that allow a cutting head of a reciprocating saw blade to engage flush with ends of the slot, thereby allowing for a repeatable cutting motion to be formed. Specifically, a reciprocating toothbrush saw can have front and rear end faces that engage with the T-shaped end slots. Thus, the saw blade head can get further posterior and anterior because there is no interference from the curved surfaces of the radiused ends.

In an example, a cutting guide for a tibial implant can comprise a plate configured to engage a resected surface of a tibia and an elongate slot extending through the plate along a slot axis, the elongate slot comprising a first longitudinal wall extending parallel to the slot axis, a second longitudinal wall extending parallel to the slot axis, and a first end wall that is flat and that extends perpendicular to the slot axis, wherein the first longitudinal wall is spaced from the second longitudinal wall by a distance and the first end wall is at least as wide as the distance.

In another example, a method of implanting a tibial tray in a knee arthroplasty can comprise resecting a proximal end of a tibia to form a proximal resected surface, positioning a plate atop the proximal resected surface, the plate including a cutting slot, positioning a cutting head through the cutting slot, advancing the cutting head in a first direction to engage flush with a flat first end of the cutting slot to form a first end of a bone channel, and positioning the tibial tray on the proximal resected surface to insert a keel into the bone channel.

In an additional example, a method of manufacturing a tibial template for a knee arthroplasty can comprise providing a tibial plate, forming a longitudinal cutting slot in the tibial plate, forming a first widening of the longitudinal cutting slot at a first end of the longitudinal cutting slot, and forming a second widening of the longitudinal cutting slot at a second end of the longitudinal cutting slot.

### BRIEF DESCRIPTION OF THE DRAWINGS

FIG. 1A is a perspective view of a knee joint in flexion having a unicondylar prosthetic device implanted therein.
FIG. 1B is a side view of the knee joint of FIG. 1A in extension.
FIG. 2A is a perspective view of the knee joint of FIG. 1A and FIG. 1B showing vertical and horizontal resections in a proximal end of the tibia.
FIG. 2B is a front view of the knee joint of FIG. 1A and FIG. 1B showing vertical and horizontal resections in the proximal end of the tibia.
FIG. 2C is a schematic side view of a tibial component of the unicondylar prosthetic device of FIG. 1A and FIG. 1B implanted in a resected tibial surface of FIG. 2A and FIG. 2B to show relative positioning between a keel and a cortical bone wall.
FIG. 3 is a perspective view of a unicondylar tibial implant of the present disclosure having a keel.
FIG. 4 is a bottom view of the unicondylar tibial implant of FIG. 4 showing the location of the keel between anterior and posterior ends of a plate.
FIG. 5 is a side view of the unicondylar tibial implant of FIG. 4.
FIG. 6 is a front view of the unicondylar tibial implant of FIG. 4.
FIG. 7 is a perspective view of a tibial template of the present disclosure including a cutting slot having widened ends.
FIG. 8 is a side view of the tibial template of FIG. 7.
FIG. 9 is a top view of the tibial template of FIG. 7.
FIG. 10 is a perspective view of a reciprocating saw blade of the present disclosure configured to interact with the cutting slot of FIG. 7.
FIG. 11 is a side view of a cutting head for the reciprocating saw blade of FIG. 10.
FIG. 12 is a top view of the cutting head of FIG. 10.
FIG. 13 is a front view of the cutting head of FIG. 10.
FIG. 14 is a schematic side view of the reciprocating saw blade of FIG. 10 seated within the tibial template of FIG. 7.
FIG. 15A is a schematic view of a tibial template of the present disclosure showing an operation of forming a cutting slot using a machining tool.
FIG. 15B is a schematic view of the tibial template of FIG. 15A showing a reciprocating saw blade head within the cutting slot.
FIG. 16A is a schematic view of a tibial template of the present disclosure showing an operation of forming T-shaped end slots using a machining tool.
FIG. 16B is a schematic view of the tibial template of FIG. 16A showing a reciprocating saw blade head within the cutting slot.
FIG. 17 is a close-up top view of a cutting slot of the present disclosure showing dimensions of the cutting slot and a widened end.
FIG. 18 is a block diagram showing operations of methods for forming a bone slot for receiving a keel of a tibial implant in a tibial bone.
FIG. 19 is a block diagram showing operations of methods for manufacturing a cutting slot in a tibial template.

### DETAILED DESCRIPTION

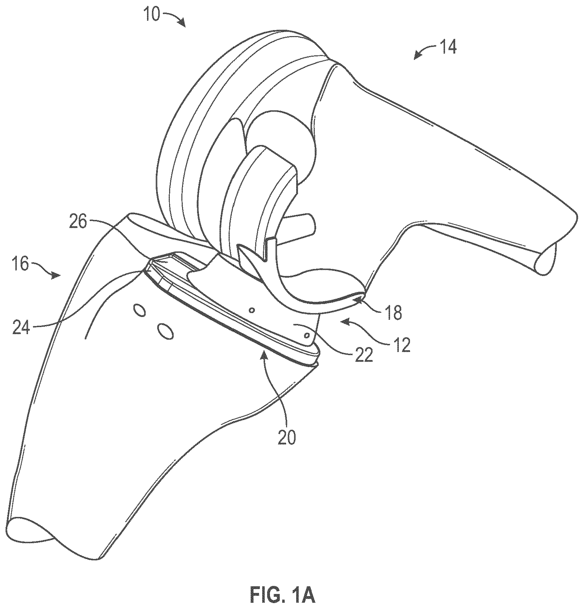

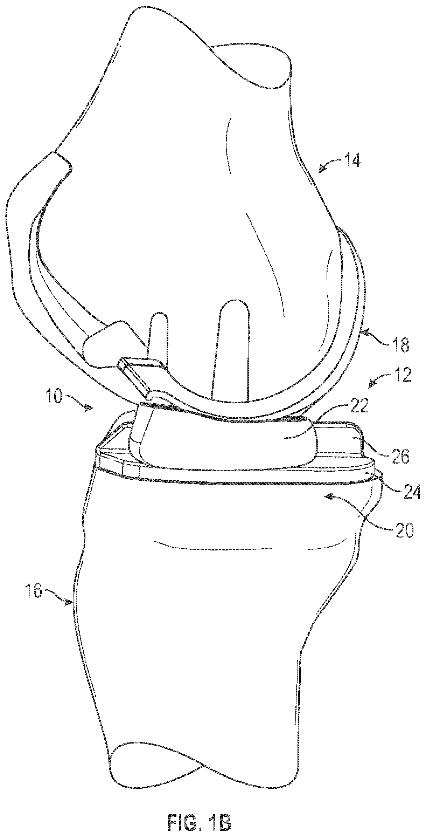

FIG. 1A is a perspective view of knee joint 10 in flexion having unicondylar prosthetic device 12 implanted therein. FIG. 1B is a side view of knee joint 10 of FIG. 1A in extension. FIG. 1A and FIG. 1B are discussed concurrently.

Knee joint 10 can comprise femur 14 and tibia 16. Unicondylar prosthetic device 12 can comprise femoral component 18 and tibial component 20. Tibial component 20 can comprise bearing insert 22.

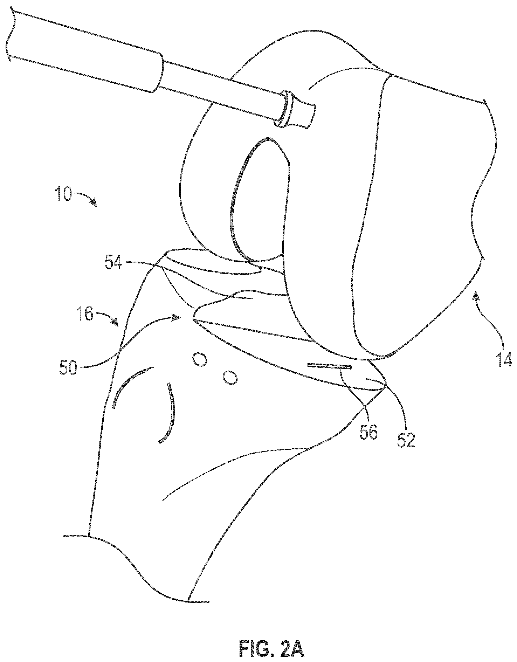

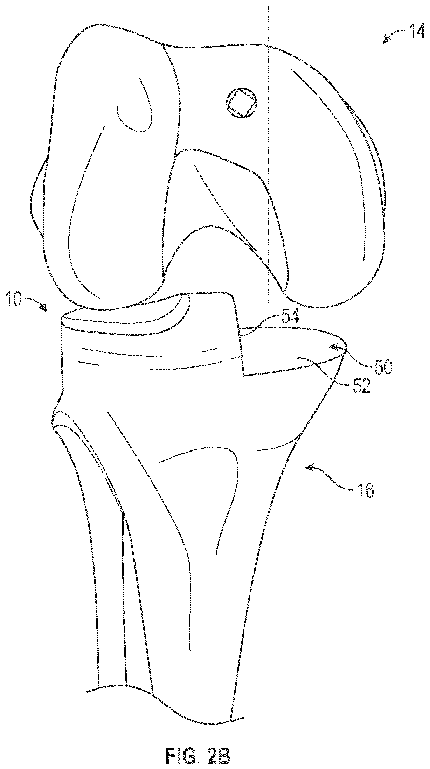

Unicondylar prosthetic device 12 can be configured to replace only one condyle of femur 14 and one condyle of tibia 16. Tibial component 20 can comprise a tray configured to receive bearing insert 22. Tibial component 20 can include plate 24 and wall 26 to engage resected bone surfaces as described with reference to FIG. 2A and FIG. 2B. Plate 24 and wall 26 can comprise planar surfaces arranged orthogonally to engage bearing insert 22. Bearing insert 22 can comprise a concave articulating surface to receive femoral component 18. Femoral component 18 can comprise a convex articulating surface to rotate against bearing insert 22.

Desirable alignment of tibial component 20 can be facilitated by medial-lateral engagement of wall 26 flush with a vertically resected bone surface of tibia 16 and when plate 24 is positioned anterior-posteriorly on a horizontally resected bone surface of tibia 16 between anterior and posterior cortical bone walls.

FIG. 2A is a perspective view of knee joint 10 of FIG. 1A and FIG. 1B showing bone modifications 50 comprising horizontal resection surface 52 and vertical resection surface 54 in a proximal end of tibia 16. FIG. 2B is a front view of knee joint 10 of FIG. 1A and FIG. 1B showing horizontal resection surface 52 and vertical resection surface 54 in the proximal end of tibia 16. FIG. 2A and FIG. 2B are discussed concurrently.

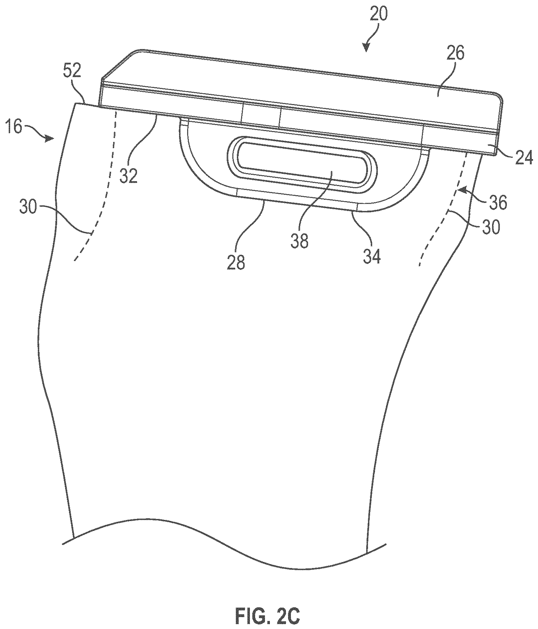

When implanted, plate 24 of tibial component 20 (FIG. 1A) can be positioned on horizontal resection surface 52 in a medial-lateral direction so that wall 26 engages with vertical resection surface 54. Additionally, tibial component 20 can be positioned in an anterior-posterior direction so that plate 24 is disposed on horizontal resection surface 52 relative to anterior and posterior cortical bone, as discussed with reference to FIG. 2C.

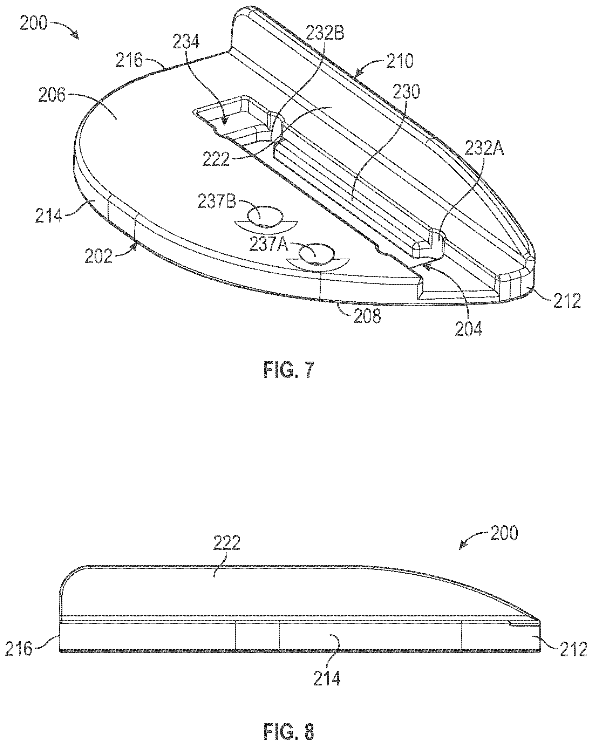

The ultimate placement of tibial component 20 on tibia 16 can be determined by positioning a tibial template, such as tibial template 200 of FIG. 7, in engagement with horizontal resection surface 52 and vertical resection surface 54 to form bone channel 56. A surgeon can slide tibial template 200 in medial-lateral direction to engage vertical resection surface 54 and then slide tibial template 200 in an anterior-posterior direction to place a cutting slot for a keel between the anterior and posterior cortical walls. A surgeon can utilize skill and experience to position tibial template 200 so that the cutting slot for the keel is placed in a location that will provide the patient with a desirable outcome.

FIG. 2C is a schematic side view of tibial component 20 of unicondylar prosthetic device 12 of FIG. 1A and FIG. 1B implanted in horizontal resection surface 52 to show the relative position between keel 28 and cortical bone matter 30. Bottom surface 32 of plate 24 of tibial component 20 can be positioned on top of horizontal resection surface 52. It can be desirable for plate 24 to rest on cortical bone matter 30 at anterior and posterior locations to facilitate support of loading placed on tibial component 20 from femur 14 (FIG. 1A). Keel 28 can be pushed below horizontal resection surface 52 into cancellous bone matter 34 between cortical bone matter 30. It can be advantageous for keel 28 to be positioned between the anterior and posterior portions of cortical bone matter 30 to avoid formation of stress concentrations on tibia 16. In particular, it can be desirable to ensure that keel 28 is positioned sufficiently forward of the posterior cortex 36 to prevent undesirable loading on tibia 16. With the present disclosure, a tibial template can be configured to provide accurate placement of keel 28 on horizontal resection surface 52 to avoid undesirable outcomes for the patient.

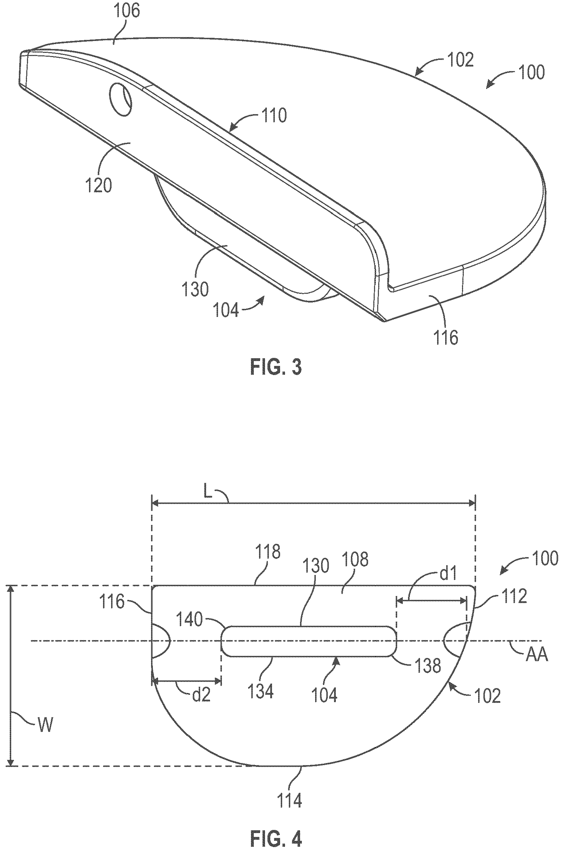

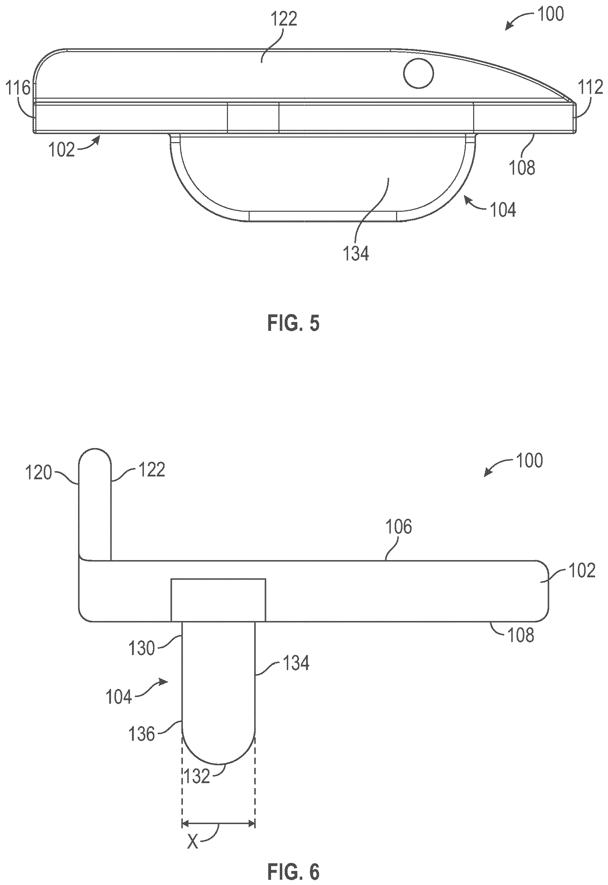

FIG. 3 is a perspective view of unicondylar tibial implant 100 of the present disclosure having baseplate 102 and keel 104. FIG. 4 is a bottom view of unicondylar tibial implant 100 of FIG. 4 showing the location of keel 104 along baseplate 102 in the anterior-posterior direction. FIG. 5 is a side view of unicondylar tibial implant 100 of FIG. 4. FIG. 6 is a front view of unicondylar tibial implant 100 of FIG. 4. FIG. 3 through FIG. 6 are discussed concurrently. In examples, unicondylar tibial implant 100 can comprise tibial component 20 of FIG. 1A, FIG. 1B and FIG. 2C.

Baseplate 102 can comprise bearing surface 106, bone-engaging surface 108 and upstanding wall 110. Baseplate 102 can further comprise anterior end 112, medial side 114, posterior end 116 and lateral side 118. Upstanding wall 110 can comprise surface 120 and surface 122. Keel 104 can comprise base 130 adjacent to bone-engaging surface 108 and tip 132 distal from base 130. Keel 104 can also include medial wall 134 and lateral wall 136.

Baseplate 102 can be configured to replace an articular surface of a tibia of a patient, such as tibia 16 of FIG. 1A. FIG. 3 through FIG. 6 illustrate a medial tibial bearing surface. The present disclosure can be applied to both medial and lateral bearing surfaces. A lateral tibial component can comprise a mirror image of the component illustrated in FIG. 3. In the example shown, baseplate 102 is generally C-shaped. In examples, baseplate 102 can have different shapes, and can for instance be generally rounded or elliptical. The shape of baseplate 102 on anterior end 112, medial side 114 and posterior end 116 can be configured to approximate a posterior and anterior shape, respectively, of an outer posterior and anterior surface, respectively, of a condyle of a tibia. For example, anterior end 112, medial side 114 and posterior end 116 can be configured to approximate the outer surface of cortical bone matter 30 of FIG. 2C.

Baseplate 102 can be installed such that bone-engaging surface 108 rests upon a surface of a tibia when the component is installed, such as horizontal resection surface 52 of FIG. 2A. Surface 120 of upstanding wall 110 can be configured to abut a central tibial eminence of a tibia when the component is installed, such as vertical resection surface 54 of FIG. 2A. Surface 122 of upstanding wall 110 can be configured to abut a bearing component, such as bearing insert 22 of FIG.1A. Upstanding wall 110 can extend upwardly from bearing surface 106 of baseplate 102 normal to the plane of baseplate 102.

Bearing surface 106 when installed will take the place of the tibial bearing surface of the anatomic tibia. Bearing surface 106 can be shaped to cooperate either directly with a condyle of a femur of a patient or a replacement prosthetic component, or with a bearing component located between unicondylar tibial implant 100 and the femoral anatomy or femoral replacement component. In examples, bearing surface 106 can be convex both anteriorly-posteriorly and laterally-medially in the case where unicondylar tibial implant 100 is for a lateral condyle. In examples, bearing surface 106 can be flat or concave in the case where unicondylar tibial implant 100 is for a medial condyle. In examples, baseplate 102 can be substantially flat, and can have an outline shaped to mimic the natural shape of the head of a tibia. Bone-engaging surface 108 of baseplate 102 can be configured to be secured to a proximal end of a tibia.

Medio-lateral width W of the baseplate 102 is defined between the medial side 114 and lateral side 118. Longitudinal antero-posterior axis AA can extends between anterior end 112 and posterior end 116. Keel 104 can extend along longitudinal anterior-posterior axis AA. In examples, longitudinal anterior-posterior axis AA can be located approximately one third of the width of the plate (i.e. 1/3W) from lateral side 118, and defines a longitudinal axis length L of baseplate 102.

Primary fixation of baseplate 102 can be provided by keel 104. Fixation is important to hold unicondylar tibial implant 100 in a fixed manner after implantation, so as to allow bone matter to grow into and attach to bone-engaging surface 108 and keel 104 to achieve secondary fixation. Keel 104 can project distally from bone-engaging surface 108. Keel 104 can comprise an elongate projection having an anterior-posterior length and a medio-lateral width. The length can be greater than the width. Keel 104 can project distally from bone-engaging surface 108 perpendicular to bone-engaging surface 108. Keel 104 can extend longitudinally in a direction generally parallel with lateral side 118 of baseplate 102. In other examples, keel 104 can project distally oblique to bone-engaging surface 108.

In examples, keel 104 can comprise a solid or continuous body between medial wall 134 and lateral wall 136, as illustrated. Such a configuration can be intended for use in a cementless fashion. In examples, cementless versions of keel 104 can be configured to be force-fit into a slot formed in bone matter of the tibia. In examples, keel 104 can comprise a slot, such as slot 38 shown in FIG. 2C, between medial wall 134 and lateral wall 136 to receive one or both of receive bone cement and bone in-growth. Cemented or cementless keels may or may not have a through-hole or slot in the keel. Cementless components can include porous coatings to encourage bony ingrowth.

Keel 104 can be located centrally on bone-engaging surface 108 with regard to anteroposterior axis AA. In particular, keel 104 can be spaced such that distance d1 between leading edge 138 of keel 104 and anterior end 112 of baseplate 102 is approximately the same as distance d2 between trailing edge 140 of keel 104 and posterior end 116 of baseplate 102. In the illustrated example, keel 104 is not located centrally with respect to width W of baseplate 102. Keel 104 can be located approximately 1/3W from lateral side 118. In examples, keel 104 can be located in other medial-lateral positions. For example, a region of baseplate 102 from which keel 104 extends can be located in an innermost half of baseplate 102 that is closest to the intercondylar eminence.

Typical dimensions for baseplate 102 can comprise AP axis length L = 44.92 mm, keel length = 25.92, mm dl = d2 = 9.50 mm, keel distance from lateral side = 1/3 W = 7.98 mm. In examples, thickness X (FIG. 6) of keel 104 can be approximately 3 mm. In examples, thickness X can be 2.0 mm+/-0.1 mm or 1.5 mm+/-0.1 mm, or 2.5 mm+/-0.1 mm. In some examples, thickness X can be between 2.4 mm and 1.7 mm, or between 1.9 mm and 1.5 mm.

As discussed, it is important for keel 104 to be positioned to avoid contacting cortical bone. A surgeon can determine the anterior-posterior position of keel 104 by placement of tibial template 200 (FIG. 7) on horizontal resection surface 52. Tibial template 200, as disclosed herein, can ensure that keel 28 (FIG. 2C) is positioned where intended by the surgeon by providing a cutting slot that allows for an accurate, repeatable cutting process.

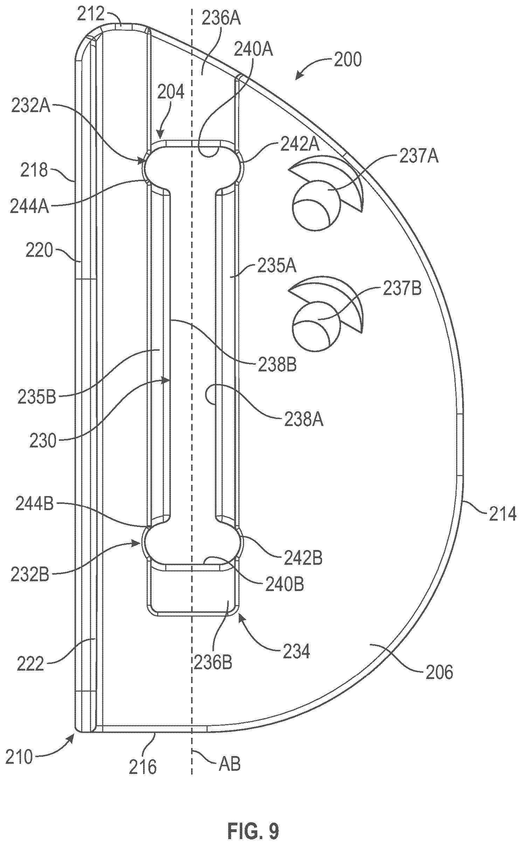

FIG. 7 is a perspective view of tibial template 200 of the present disclosure comprising plate 202 and cutting slot 204. FIG. 8 is a side view of tibial template 200 of FIG. 7. FIG. 9 is a top view of tibial template 200 of FIG. 7. FIG. 7, FIG. 8 and FIG. 9 are discussed concurrently.

Plate 202 can comprise bearing surface 206, bone-engaging surface 208 and upstanding wall 210. Plate 202 can further comprise anterior end 212, medial side 214, posterior end 216 and lateral side 218. Upstanding wall 210 can comprise surface 220 and surface 222. Cutting slot 204 having elongate portion 230, widened end 232A and widened end 232B. Cutting slot 204 can be located in groove 234. Groove 234 can include first rail 235A and second rail 235B, anterior pocket 236A and posterior pocket 236B. Plate 202 can additionally include first bore 237A and second bore 237B.

As best seen in FIG. 9, elongate portion 230 can include first longitudinal wall 238A and second longitudinal wall 238B, widened end 232A can include anterior end wall 240A, and widened end 232B can include posterior end wall 240B. Arcuate wall 242A can connect first longitudinal wall 238A and anterior end wall 240A. Arcuate wall 244A can connect second longitudinal wall 238B and anterior end wall 240A. Arcuate wall 242B can connect first longitudinal wall 238A and posterior end wall 240B. Arcuate wall 244B can connect second longitudinal wall 238B and posterior end wall 240B. In examples, anterior end wall 240A and posterior end wall 24B can be disposed at ninety-degrees to first longitudinal wall 238A and second longitudinal wall 238B. In examples, anterior end wall 240A and posterior end wall 24B can be centered on axis AB.

Tibial template 200 can be configured to approximate the size and shape of baseplate 102 and upstanding wall 110 of unicondylar tibial implant 100 (FIG. 3 through FIG. 6). As such, bearing surface 206, bone-engaging surface 208, upstanding wall 210, anterior end 212, medial side 214, posterior end 216, lateral side 218, surface 220 and surface 222 can be configured to be the same or nearly the same as bearing surface 106, bone-engaging surface 108, upstanding wall 110, anterior end 112, medial side 114, posterior end 116, lateral side 118, surface 120 and surface 122. However, bearing surface 206 can be advantageously configured as a flat or planar surface to facilitate the inclusion of cutting slot 204 and groove 234.

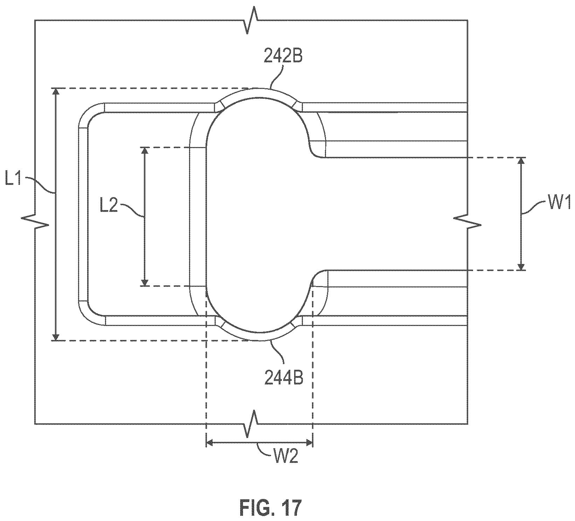

First longitudinal wall 238A and second longitudinal wall 238B can extend in linear, parallel fashion along anterior-posterior axis AB. First longitudinal wall 238A and second longitudinal wall 238B can extend all the way to widened end 232A and widened end 232B. Anterior end wall 240A and posterior end wall 240B can extend in linear, parallel fashion perpendicularly to anterior-posterior axis AB. Anterior end wall 240A can have a width relative to anterior-posterior axis AB that is at least as wide as the distance between first longitudinal wall 238A and second longitudinal wall 238B, e.g., width W1 of FIG. 17. Thus, if first longitudinal wall 238A and second longitudinal wall 238B were extended forward and backward, they would intersect anterior end wall 240A and posterior end wall 240B. In examples, arcuate wall 242A, arcuate wall 242B, arcuate wall 244A and arcuate wall 244B can comprise semi-circles. Thus, for example, if first longitudinal wall 238A were extended forward, first longitudinal wall 238A would intersect both ends of the semi-circle of arcuate wall 242A. In examples, arcuate wall 242A and 244A can comprise semi-circles spaced further apart from each other than what is illustrated in FIG. 9 so that short segments of flat walls can connect arcuate wall 242A with first longitudinal wall 238A and anterior end wall 240A, and arcuate wall 244A with second longitudinal wall 238B and anterior end wall 240A, respectively. In examples, arcuate wall 242A, arcuate wall 242B, arcuate wall 244A and arcuate wall 244B can have other curved shapes than semi-circular.

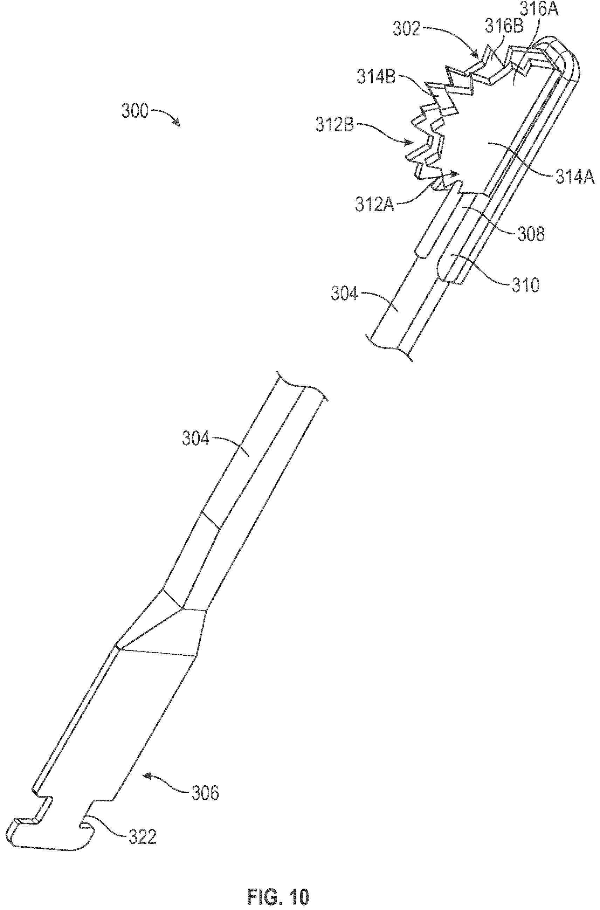

Tibial template 200 can be used determine the location for keel 104 in horizontal resection surface 52 (FIG. 1A) and form a slot within horizontal resection surface 52 for keel. Specifically, a surgeon can position cutting slot 204 on horizontal resection surface 52 at a desired location and then insert a cutting tool, such as reciprocating saw blade 300 of FIG. 10, into cutting slot 204. Thus, a surgeon can position the anterior end of plate 202 proximate the anterior portion of horizontal resection surface 52 and the posterior end of plate 202 proximate the posterior portion of horizontal resection surface 52. One or both of first bore 237A and second bore 237B can be used to immobilize tibial template 200 relative to the tibia, such as by inserting a pin or nail therethrough into bone matter.

As discussed in greater detail below, cutting slot 204 and groove 234 can cooperate to receive reciprocating saw blade 300 of FIG. 10. Widened end 232A and widened end 232B can include flat posterior and anterior surfaces, anterior end wall 240A and posterior end wall 240B, respectively, to allow reciprocating saw blade 300 to advance fully forward and backward to form a bone slot or channel having flat or planar anterior and posterior faces and that has a precise length that allow keel 104 (FIG. 3) to be positioned fully anteriorly or posteriorly, depending on surgeon placement and other factors.

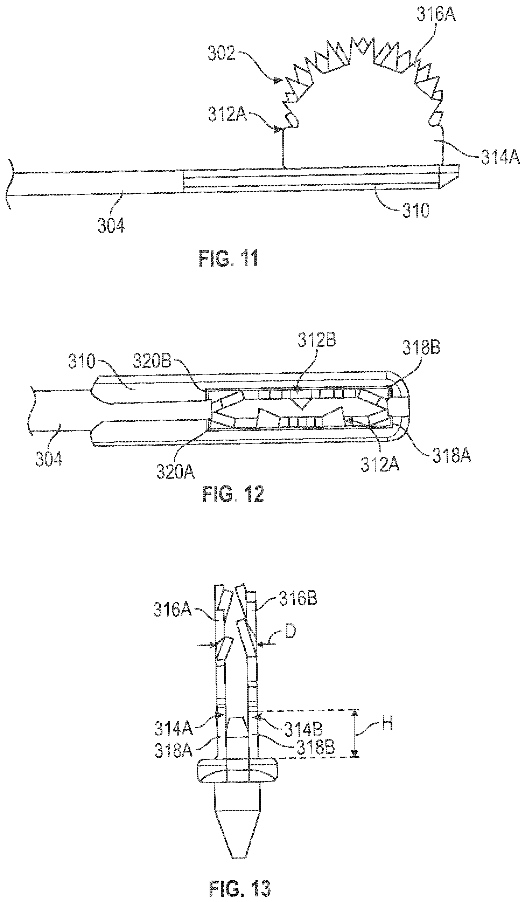

FIG. 10 is a perspective view of reciprocating saw blade 300 of the present disclosure configured to interact with cutting slot 204 of FIG. 7. Reciprocating saw blade 300 can comprise cutting head 302, shaft 304 and coupler 306. Cutting head 302 can comprise tip 308, shoulder 310, first blade 312A and second blade 312B. First blade 312A can comprise first plate portion 314A and a plurality of first teeth 316A. Second blade 312B can comprise second plate portion 314B and a plurality of second teeth 316B. FIG. 11 is a side view of cutting head 302 for reciprocating saw blade 300 of FIG. 10. FIG. 12 is a top view of cutting head 302 of FIG. 10. FIG. 13 is a front view of cutting head 302 of FIG. 10. FIG. 10 through FIG. 12 are discussed concurrently.

Coupler 306 can be configured to connect to a power source, such as a hand tool. In examples, coupler 306 can be configured to connect to a reciprocating mechanism of a reciprocating power tool. In examples, coupler 306 can comprise a flat portion having notch 322 onto which a clamping mechanism or a chuck of a power tool can attach to. Shaft 304 can extend from coupler 306 and can allow cutting head 302 to be extended into anatomy, such as a knee joint, to perform a cutting operation. Reciprocating saw blade 300 can be fabricated from a rigid material, such as stainless steel, to facilitate transmission of a reciprocating input to cutting head 302 while minimizing bending, vibration and the like.

Shoulder 310 can comprise a base from which first blade 312A and second blade 312B extend. First plate portion 314A and second plate portion 314B can extend from shoulder 310 parallel to each other and perpendicular to shoulder 310. First plate portion 314A can include planar end face 318A and planar end face 320A. Second plate portion 314B can include planar end face 318B and planar end face 320B. First plate portion 314A and second plate portion 314B can extend parallel to each other. First plate portion 314A and second plate portion 314B can have height H (FIG. 13) above shoulder 310. First plate portion 314A and second plate portion 314B can be spaced apart from each other such that exterior, e.g., outwardly facing surfaces, can be distance D (FIG. 13) away from each other. In examples, distance D can be equal to thickness X of keel 104 (FIG. 6). The plurality of first teeth 316A and the plurality of second teeth 316B can be configured to extend distally from shoulder 310 a predetermined amount that can be designed to suit keel 104.

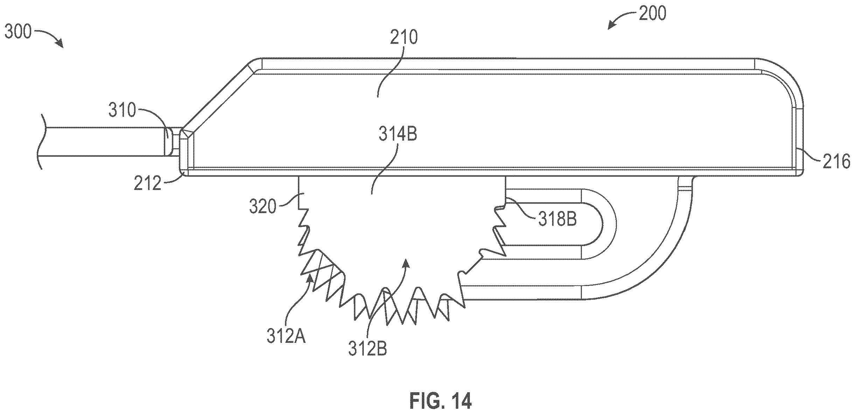

Reciprocating saw blade 300 can be configured to engage with cutting slot 204 and groove 234 (FIG. 7) to form a rectilinear slot or channel, wherein the length of the slot can be consistently produced, within horizontal resection surface 52 (FIG. 2A), as discussed in greater detail with reference to FIG. 14.

FIG. 14 is a schematic side view of reciprocating saw blade 300 of FIG. 10 seated within tibial template 200 of FIG. 7. Reciprocating saw blade 300 can interact with tibial template 200 to control movements of cutting head 302 relative to plate 202. Reciprocating saw blade 300 can be configured to approach tibial template 200 from the anterior direction so that cutting head 302 extends past anterior end 212 to reach toward posterior end 216. Shoulder 310 can be configured to fit within groove 234 such that shoulder 310 can ride on first rail 235A and second rail 235B alongside first blade 312A and second blade 312B. Tip 308 can sit atop posterior pocket 236B and shoulder 310 can extend along anterior pocket 236A. Posterior pocket 236B can be axial longer than tip 308 to allow cutting head 302 to be able to advance fully posteriorly so that planar end face 318A and planar end face 318B engage posterior end wall 240B. Shaft 304 can extend from shoulder 310 through anterior pocket 236A. The plurality of first teeth 316A and the plurality of second teeth 316B penetrate through plate 202 so that first plate portion 314A and second plate portion 314B engage with the wall of cutting slot 204.

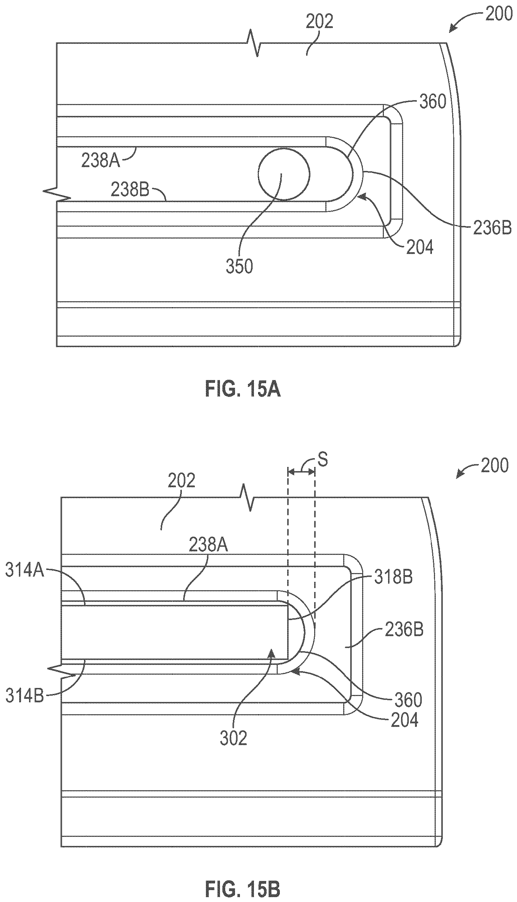

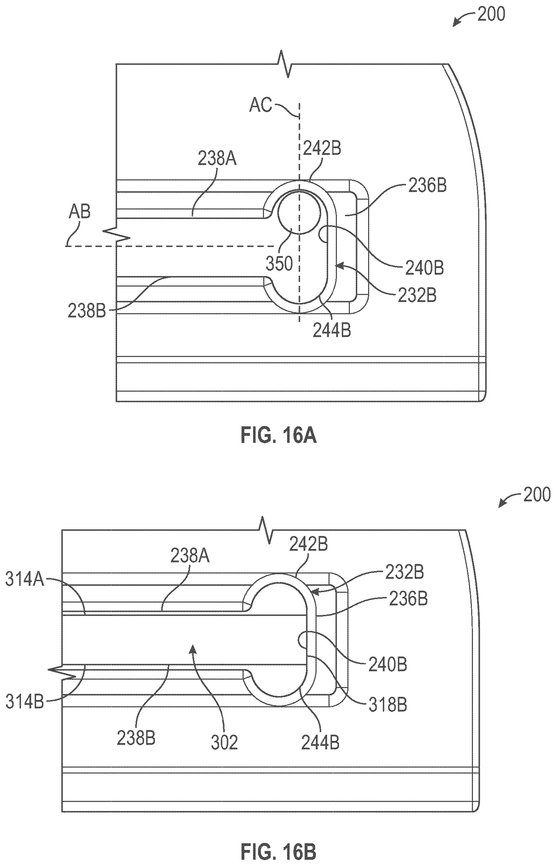

First plate portion 314A can extend along first longitudinal wall 238A. Second plate portion 314B can extend along second longitudinal wall 238B. Planar end face 318A and planar end face 318B can face toward and engage flush with posterior end wall 240B as cutting head 302 is reciprocated. Planar end face 320A and planar end face 320B can face toward and engage flush with anterior end wall 240A as cutting head is reciprocated. As discussed with reference to FIG. 15B and FIG. 16B, planar end face 318B can engage flush with posterior end wall 240B to allow for cutting head 302 to consistently cut a slot into bone matter without interference, e.g., binding, between cutting head 302 and cutting slot 204.

FIG. 15A is a schematic view of tibial template 200 showing machining tool 350 within elongate portion 320 of cutting slot 204. Tibial template 200 can comprise plate 202 in which cutting slot 204 extends. Cutting slot 204 can extend along an axis and can have first longitudinal wall 238A and second longitudinal wall 238B. However, contrary to the present disclosure, first longitudinal wall 238A and second longitudinal wall 238B can be connected by arcuate end wall 360. FIG. 15A shows an example of an operation in the formation of cutting slot 204, wherein elongate portion 320 has been fabricated, but widened end 232A and widened end 232B have not yet been formed. FIG. 15A only shows one end of cutting slot 204, but the opposite end can be formed in a similar manner.

Machining tool 350 can comprise a circular shaped cutting body, such as a milling cutter including a ball nose end mill cutter, a square end mill cutter, a radius end milling cutter, a corner radius end milling cutter, and the like. Because these milling cutters can have circular cutting profiles, as shown in FIG. 15A, they typically only produce straight, flat or planar surfaces when moved along an axis. As such, first longitudinal wall 238A and second longitudinal wall 238B can be made by moving machining tool 350 along one or more paths parallel to cutting slot 204. However, when machining tool 350 reaches the end of cutting slot 204, arcuate end wall 360 can be produced instead of a flat wall.

FIG. 15B is a schematic view of tibial template 200 of FIG. 15A showing cutting head 302 within cutting slot 204. FIG. 15B can illustrate a hypothetical engagement of cutting head 302 with elongate portion 320 before the formation of widened end 232A and widened end 232B. Due to the presence of arcuate end wall 360, cutting head 302 is prevented from reaching the furthest-most end of cutting slot 204 by distance S. Arcuate end wall 360 prevents planar end face 318B of cutting head 302 from advancing fully to the right (relative to the orientation of FIG. 15B) equal to distance S. The present inventors have recognized that the presence of distance S can produce deleterious results in the placement of keel 104 (FIG. 3) within horizontal resection surface 52 (FIG. 2A). For example, the presence of arcuate end wall 360 at the posterior end of cutting slot 204 can result in keel 104 be placed too far anteriorly. Similarly, the presence of an arcuate end wall similar to arcuate end wall 360 at the anterior end of cutting slot 204 can result in keel 104 be placed too far posteriorly. As such, this variability in the actual placement of the bone channel or slot formed by cutting head 302, e.g., bone channel 56 of FIG. 2A) due to curved end walls can result in keel coming too close to or even into contact with the posterior cortex of the tibia, which can place undesirable stress on the bone matter that can in extreme cases lead to fracture of bone matter.

FIG. 16A is a schematic view of tibial template 200 of the present disclosure showing machining tool 350 within cutting slot 204 of the present disclosure. First longitudinal wall 238A and second longitudinal wall 238B can be made by moving machining tool 350 along one or more paths parallel to cutting slot 204, as shown in FIG. 15A. However, when machining tool 350 reaches the end of cutting slot 204, an arcuate end wall can be produced, similar to arcuate end wall 360 of FIG. 15A. However, instead of leaving the arcuate end wall in the finished product, machining tool 350 can be moved along additional paths to form widened end 232B. Note, widened end 232A (FIG. 9) can be fabricated by the same process as widened end 232B. Widened end 232B can be formed by moving machining tool 350 along a path perpendicular to elongate portion 230 and axis AB one or more times. Machining tool 350 can be moved along axis AC. In examples, machining tool 350 can be moved along axis AC to position posterior end wall 240B where the apex of arcuate end wall 360 is located. As such, the overall longitudinal length of cutting slot 204 in FIG. 15A and FIG. 16A can be the same. However, in additional examples, FIG. 15A can represent an intermediate step in the formation of the overall longitudinal length of cutting slot 204 such that the formation of one or both of widened end 232A and widened end 232B can elongate or lengthen cutting slot 204 from what is formed in FIG. 15A. FIG. 16A shows an example of an operation in the formation of cutting slot 204, wherein elongate portion 320 and widened end 232A and widened end 232B are formed.

FIG. 16B is a schematic view of tibial template 200 of FIG. 16A showing cutting head 302 within cutting slot 204. Posterior end wall 240B can allow planar end face 318B of cutting head 302 to advance fully to the right (relative to the orientation of FIG. 16B), i.e., such that posterior end wall 240B and cutting head 302 are engaged flush. As compared to FIG. 15B, cutting head 302 can be advanced a distance further to the right an amount equal to distance S. As such, keel 104 can be placed within the cut bone slot is a position selected by a surgeon, such as a position to avoid contact with cortical bone. Furthermore, the width of cutting head 302, e.g., distance D of FIG. 13, is irrelevant to the anterior-posterior formation of the bone channel (e.g., bone channel 56 of FIG. 2A) because anterior end wall 240A and posterior end wall 240B cutting slot 204 are flat. For example, if a cutting head that is narrower than cutting head 302 were to be used, such a cutting head could advance further anteriorly or posteriorly with a radiused cutting slot (similar to what is shown in FIG. 15A) because the radiused end will have less impact on a narrow cutting head. The present disclosure can eliminate this variable from the bone channel formation process.

FIG. 17 is a close-up top view of cutting slot 204 of the present disclosure showing dimensions of elongate portion 230 and widened end 232B. Elongate portion 230 can have width W1. Widened end 232B can have overall length L1, flat length L2 and width W2. L1 is greater than L2. In examples, L2 can be equal to or greater than W1. Flat length L2 can be equal to or greater than distance D (FIG. 13) between the outsides of first plate portion 314A and second plate portion 314B. Length L1 can be sufficiently large to produce flat length L2 to be equal to or greater than width W1. Length L1 can be determined based on the cutting diameter of machining tool 350. For example, the difference between L1 and L2 can be equal to the diameter of machining tool 350, with half of the diameter being on either side of flat length L2. However, L1 can be greater in length than the length of L2 plus the diameter of machining tool 350.

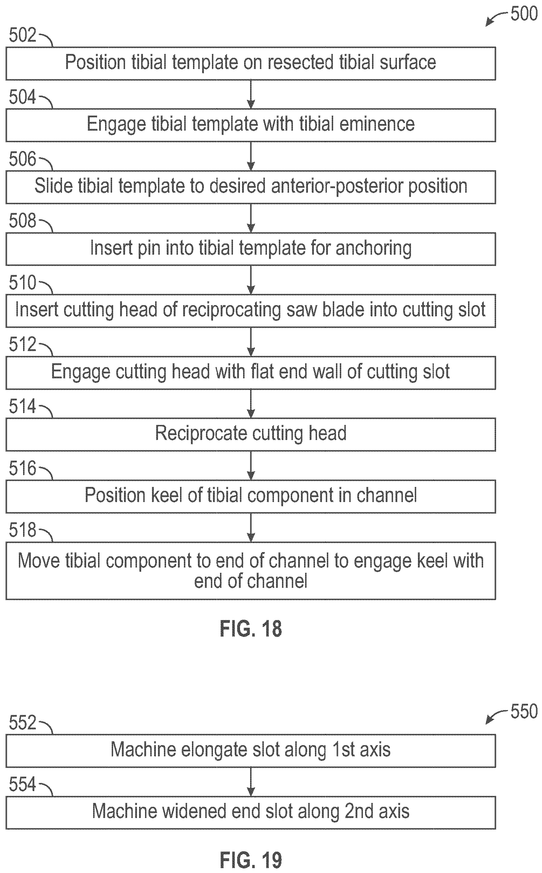

FIG. 18 is a block diagram showing operations of method 500 for forming a slot in bone for keel 104 of unicondylar tibial implant 100. Though discussed with reference to unicondylar tibial implant 100 and tibial template 200, method 500 can encompass the use of similarly configured devices. Method 500 can additionally include fewer or greater operations other than operation 502 to operation 518. Additionally, in other examples, operation 502 through operation 518 can be performed in other sequences.

At operation 502, tibial template 200 can be positioned on horizontal resection surface 52. A surgeon can position the tibial template on the horizontal resection surface of the tibia. The tibial template is designed to approximate the size and shape of the baseplate of the tibial implant and includes a cutting slot with widened ends to accommodate the saw blade.

At operation 504, tibial template 200 can be engaged with a tibial eminence formed at vertical resection surface 54. The tibial template can be slid along the horizontal resection surface to engage vertical resection surface, thereby locating the tibial template in the medial-lateral direction.

At operation 506, tibial template 200 can be slid along horizontal resection surface 52 in an anterior-posterior direction until cutting slot 204 is in a desired location. For example, the tibial template can be slid so that the anterior end is aligned with an anterior cortical bone wall, or the tibial template can be slid so that the posterior end is aligned with a posterior cortical bone wall. In examples, cutting slot 204 can be positioned to be between an anterior cortical bone wall and a posterior cortical bone wall.

At operation 508, a pin can be inserted into tibial template 200, such as at first bore 237A or second bore 237B, to immobilize tibial template 200 on horizontal resection surface 52. The tibial template is secured in place on the horizontal resection surface to ensure that the template does not move during the cutting process. This can be done by inserting a pin through the template and into bone matter of the tibia. The pin can prevent anterior-posterior movement of the tibial template and can wedge the tibial template against the tibial eminence.

At operation 510, cutting head 302 of reciprocating saw blade 300 can be inserted into cutting slot 204. A reciprocating saw blade specifically designed to interact with the tibial template can be is inserted into the cutting slot. The saw blade can have a cutting head with shoulders configured to engage rails alongside the cutting slot, and blades with teeth configured to extend through the tibial template to cut bone matter. The blades can be spaced to match the width of the keel and the cutting slot. The blades can have flat end faces to engage with flat end walls of widened ends of the cutting slot.

At operation 512, cutting head 302 can be engaged with widened end 232A and widened end 232B. Specifically, planar end face 318A and planar end face 318B can be engaged with posterior end wall 240B and planar end face 320A and planar end face 320B can be engaged with anterior end wall 240A. The surgeon can move cutting head 302 to engage planar end face 318A and planar end face 318B.

At operation 514, cutting head 302 can be reciprocated within cutting slot 204, such as by a power tool attached to coupler 306. Additionally, a user or operator of reciprocating saw blade 300 can move cutting head 302 in a so-call dolphin pattern, e.g., along a sin wave, within cutting slot 204 while the power tool is reciprocating cutting head 302. The width of the cut slot int the bone can be determined by the distance between the longitudinal walls of the cutting slot, and the length of the cut slot in the bone can be controlled by the engagement of the saw blade with the flat end walls of the widened ends. Cutting head 302 can be moved back and forth by a surgeon in a macro fashion simultaneously while a power tool reciprocates cutting head 302 back and forth in a micro fashion. Thus, the surgeon can form the bone channel, e.g., bone channel 56 of FIG. 2A, by engaging cutting head 302 with planar end face 318A and planar end face 318B, while the reciprocation from the power tool can provide the bone cutting action. In examples, operation 512 and operation 514 can be performed simultaneously.

At operation 516, keel 104 of unicondylar tibial implant 100 can be positioned within the channel or slot formed by plurality of first teeth 316A and plurality of second teeth 316B of cutting head 302. The surgeon can position keel 104 in a desired location to position unicondylar tibial implant 100 on tibia 16 to provide support with cortical bone matter 30 and to provide alignment with femoral component 18. Furthermore, the surgeon can position keel 104 away from contact with cortical bone matter 30.

At operation 518, unicondylar tibial implant 100 can be moved within the channel or slot formed by cutting head 302, such as to engage leading edge 138 of keel 104 with the anterior end of the channel or slot formed by cutting head 302. To ensure that keel 104 is not engaged with cortical bone matter 30 in one direction, the surgeon can push keel 104 into engagement with the end of the bone slot in the opposite direction. Operation 518 can be performed optionally. For example, keel 104 can be positioned to be between ends of the bone slot formed by cutting slot 204.

FIG. 19 is a block diagram showing operations of method 550 for forming cutting slot 204 in tibial template 200. Though discussed with reference to tibial template 200, method 550 can encompass the use of similarly configured devices. Method 550 can additionally include fewer or greater operations other than operation 552 to operation 554. Additionally, in other examples, operation 552 through operation 554 can be performed in other sequences.

At operation 552, elongate portion 230 can be formed in plate 202 of tibial template 200. Elongate portion 230 can be formed by moving a milling tool, e.g., machining tool 350, along axis AB. Plate 202 can be held stationary in a fixture or jig. Elongate portion 230 can be formed to have the length that is at least as long as the length of keel 104.

At operation 554, widened end 232A and widened end 232B can be formed at ends of elongate portion 230. Widened end 232A and widened end 232B can be formed by moving a milling tool, e.g., machining tool 350, perpendicular to elongate portion 320, such as along axis AC of FIG. 16A. Widened end 232A and widened end 232B can increase the length of elongate portion 230 formed at operation 552 or can only widen the ends of elongate portion 230.

As discussed herein, it can be difficult to achieve flush engagement of a cutting head of a toothbrush type cutting blade with ends of a slot. Typically there is a radius at the corners of the slot that is a result of a machining process, such as a milling process wherein a rotating cutting bit having a radius is used to make an elongate slot. The radius can be problematic because the saw blade can bind up in the corner of the guide slot. Thus, the precise placement of the bone channel produced with the cutting head can vary from procedure to procedure even though the same template and same cutting head are used. With the present disclosure, a cutting slot having T-shaped ends can be used to allow the cutting head to advance to a repeatable location without interference from the cutting slot, such as binding with the radius at each corner. The T-shape allows for a controllable and repeatable process to be performed so that the bone channel is finally produced where intended by the surgeon. The T-shape can allow ninety-degree cuts to be made, such as by allowing front and rear faces of the cutting head above the cutting teeth of the cutting head to hit the slot. As such, the bone channel can be placed where intended by the surgeon to be away from cortical bone matter, particularly on the posterior side, thereby reducing the risk of fracture. Furthermore, with the present disclosure the width of the cutting head, e.g., distance D of FIG. 13, is irrelevant to the anterior-posterior formation of the bone channel because the flat ends of the cutting slot are flat. Thus, the present disclosure can eliminate this variable from the bone channel formation process.

### Examples

Example 1 is a cutting guide for a tibial implant, the cutting guide comprising: a plate configured to engage a resected surface of a tibia; and an elongate slot extending through the plate along a slot axis, the elongate slot comprising: a first longitudinal wall extending parallel to the slot axis; a second longitudinal wall extending parallel to the slot axis; and a first end wall that is flat and that extends perpendicular to the slot axis; wherein: the first longitudinal wall is spaced from the second longitudinal wall by a distance; and the first end wall is at least as wide as the distance.

In Example 2, the subject matter of Example 1 optionally includes wherein the first end wall forms part of a first widened end of the elongate slot, the first widened end having a length greater than the distance.

In Example 3, the subject matter of any one or more of Examples 1-2 optionally include wherein the first widened end of the elongate slot comprises a first T-shaped end of the elongate slot.

In Example 4, the subject matter of Example 3 optionally includes wherein the first T-shaped end is centered on the elongate slot.

In Example 5, the subject matter of any one or more of Examples 3-4 optionally include a second T-shaped end of the elongate slot, the second T-shaped end comprising a second end wall disposed perpendicular to the first longitudinal wall and the second longitudinal wall.

In Example 6, the subject matter of Example 5 optionally includes wherein the elongate slot comprises a length and the length is greater than the distance.

In Example 7, the subject matter of any one or more of Examples 5-6 optionally include wherein: first T-shaped end of the elongate slot comprises: a first arcuate end connecting the first longitudinal wall of the elongate slot and the first end wall; and a second arcuate end connecting the second longitudinal wall of the elongate slot and the first end wall; and second T-shaped end of the elongate slot comprises: a third arcuate end connecting the second longitudinal wall of the elongate slot and the second end wall; and a fourth arcuate end connecting the second longitudinal wall of the elongate slot and the second end wall.

In Example 8, the subject matter of any one or more of Examples 5-7 optionally include wherein the elongate slot, the first T-shaped end and the second T-shaped end are located within a groove in the plate.

In Example 9, the subject matter of Example 8 optionally includes wherein the groove comprises: a posterior pocket located posterior of the elongate slot; an anterior pocket located anterior of the elongate slot; and a pair of rails extending alongside opposite sides of the elongate slot.

In Example 10, the subject matter of Example 9 optionally includes wherein the anterior pocket extends through a perimeter of the plate.

In Example 11, the subject matter of any one or more of Examples 1-10 optionally include one or more bores extending through the plate for receiving an anchoring pin.

In Example 12, the subject matter of any one or more of Examples 1-11 optionally include wherein the plate comprises a tibial template for a partial knee arthroplasty, the plate comprises an upstanding, vertical wall extending perpendicular to the plate.

In Example 13, the subject matter of Example 12 optionally includes wherein the plate comprises: a straight edge along which the upstanding, vertical wall extends; and a curved edge disposed opposite the straight edge to approximate a curvature of a tibia.

Example 14 is a method of implanting a tibial tray in a knee arthroplasty, the method comprising: resecting a proximal end of a tibia to form a proximal resected surface; positioning a plate atop the proximal resected surface, the plate including a cutting slot; positioning a cutting head through the cutting slot; advancing the cutting head in a first direction to engage flush with a flat first end of the cutting slot to form a first end of a bone channel; and positioning the tibial tray on the proximal resected surface to insert a keel into the bone channel.

In Example 15, the subject matter of Example 14 optionally includes advancing the cutting head in a second direction opposite the first direction to engage flush with a flat second end of the cutting slot to form a second end of the bone channel.

In Example 16, the subject matter of Example 15 optionally includes positioning the keel between the first end and the second end of the bone channel to avoid contact with cortical bone wall.

In Example 17, the subject matter of any one or more of Examples 15-16 optionally include extending a pin through the plate to immobilize the plate on the proximal resected surface.

Example 18 is a method of manufacturing a tibial template for a knee arthroplasty, the method comprising: providing a tibial plate; forming a longitudinal cutting slot in the tibial plate; forming a first widening of the longitudinal cutting slot at a first end of the longitudinal cutting slot; and forming a second widening of the longitudinal cutting slot at a second end of the longitudinal cutting slot.

In Example 19, the subject matter of Example 18 optionally includes wherein: forming the longitudinal cutting slot in the tibial plate comprises moving a rotating machining tool along a cutting axis to form opposing longitudinal surfaces; forming the first widening of the longitudinal cutting slot at the first end of the longitudinal cutting slot comprises moving the rotating machining tool along a first axis perpendicular to the cutting axis across the opposing longitudinal surfaces of the longitudinal cutting slot; and forming the second widening of the longitudinal cutting slot at the first end of the longitudinal cutting slot comprises moving the rotating machining tool along a second axis perpendicular to the cutting axis across the opposing longitudinal surfaces of the longitudinal cutting slot.

In Example 20, the subject matter of Example 19 optionally includes wherein: moving the rotating machining tool along the first axis perpendicular to the cutting axis across the opposing longitudinal surfaces of the longitudinal cutting slot comprises removing a first rounded surface of the first end of the longitudinal cutting slot formed by the rotating machining tool; and moving the rotating machining tool along the second axis perpendicular to the cutting axis across the opposing longitudinal surfaces of the longitudinal cutting slot comprises removing a second rounded surface of the second end of the longitudinal cutting slot formed by the rotating machining tool.

Each of these non-limiting examples can stand on its own, or can be combined in various permutations or combinations with one or more of the other examples.

### Various Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A cutting guide for a tibial implant, the cutting guide comprising:
a plate configured to engage a resected surface of a tibia; and
an elongate slot extending through the plate along a slot axis, the elongate slot comprising:
a first longitudinal wall extending parallel to the slot axis;
a second longitudinal wall extending parallel to the slot axis; and
a first end wall that is flat and that extends perpendicular to the slot axis;
wherein:
the first longitudinal wall is spaced from the second longitudinal wall by a distance; and
the first end wall is at least as wide as the distance.

2. The cutting guide of claim 1, wherein the first end wall forms part of a first widened end of the elongate slot, the first widened end having a length greater than the distance.

3. The cutting guide of claim 1, wherein the first widened end of the elongate slot comprises a first T-shaped end of the elongate slot.

4. The cutting guide of claim 3, wherein the first T-shaped end is centered on the elongate slot.

5. The cutting guide of claim 3, further comprising:
a second T-shaped end of the elongate slot, the second T-shaped end comprising a second end wall disposed perpendicular to the first longitudinal wall and the second longitudinal wall.

6. The cutting guide of claim 5, wherein the elongate slot comprises a length and the length is greater than the distance.

7. The cutting guide of claim 5, wherein:
first T-shaped end of the elongate slot comprises:
a first arcuate end connecting the first longitudinal wall of the elongate slot and the first end wall; and
a second arcuate end connecting the second longitudinal wall of the elongate slot and the first end wall; and
second T-shaped end of the elongate slot comprises:
a third arcuate end connecting the second longitudinal wall of the elongate slot and the second end wall; and
a fourth arcuate end connecting the second longitudinal wall of the elongate slot and the second end wall.

8. The cutting guide of claim 5, wherein the elongate slot, the first T-shaped end and the second T-shaped end are located within a groove in the plate.

9. The cutting guide of claim 8, wherein the groove comprises:
a posterior pocket located posterior of the elongate slot;
an anterior pocket located anterior of the elongate slot; and
a pair of rails extending alongside opposite sides of the elongate slot.

10. The cutting guide of claim 9, wherein the anterior pocket extends through a perimeter of the plate.

11. The cutting guide of claim 1, further comprising one or more bores extending through the plate for receiving an anchoring pin.

12. The cutting guide of claim 1, wherein the plate comprises a tibial template for a partial knee arthroplasty, the plate comprises an upstanding, vertical wall extending perpendicular to the plate.

13. The cutting guide of claim 12, wherein the plate comprises:
a straight edge along which the upstanding, vertical wall extends; and
a curved edge disposed opposite the straight edge to approximate a curvature of a tibia.

14. A method of manufacturing a tibial template for a knee arthroplasty, the method comprising:
providing a tibial plate;
forming a longitudinal cutting slot in the tibial plate;
forming a first widening of the longitudinal cutting slot at a first end of the longitudinal cutting slot; and
forming a second widening of the longitudinal cutting slot at a second end of the longitudinal cutting slot.

15. The method of claim 14, wherein:
forming the longitudinal cutting slot in the tibial plate comprises moving a rotating machining tool along a cutting axis to form opposing longitudinal surfaces;
forming the first widening of the longitudinal cutting slot at the first end of the longitudinal cutting slot comprises moving the rotating machining tool along a first axis perpendicular to the cutting axis across the opposing longitudinal surfaces of the longitudinal cutting slot; and
forming the second widening of the longitudinal cutting slot at the first end of the longitudinal cutting slot comprises moving the rotating machining tool along a second axis perpendicular to the cutting axis across the opposing longitudinal surfaces of the longitudinal cutting slot;
optionally wherein:
moving the rotating machining tool along the first axis perpendicular to the cutting axis across the opposing longitudinal surfaces of the longitudinal cutting slot comprises removing a first rounded surface of the first end of the longitudinal cutting slot formed by the rotating machining tool; and
moving the rotating machining tool along the second axis perpendicular to the cutting axis across the opposing longitudinal surfaces of the longitudinal cutting slot comprises removing a second rounded surface of the second end of the longitudinal cutting slot formed by the rotating machining tool.
